(19) Europäisches Patentamt
European Patent Office
Office européen des brevets

(11) EP 0 955 797 B1

(12) # EUROPEAN PATENT SPECIFICATION

(45) Date of publication and mention of the grant of the patent:
**22.06.2005 Bulletin 2005/25**

(21) Application number: **97921128.1**

(22) Date of filing: **09.04.1997**

(51) Int Cl.[7]: **A01C 1/06**, A01N 25/26, C07C 273/00, C07C 275/12, C07C 269/00, C07C 271/10, C07C 275/50, A01N 47/34

(86) International application number:
**PCT/US1997/005887**

(87) International publication number:
**WO 1997/037524 (16.10.1997 Gazette 1997/44)**

(54) **DIFORMYLUREA AND REACTION PRODUCTS OF UREA AND CARBOXYLIC ACIDS**

Diformylharnstoff und Reaktionsprodukte von Harnstoff und Carbonsäuren

DIFORMYLUREE ET PRODUIT DE REACTION DE L'UREE ET D'ACIDES CARBOXYLIQUES

(84) Designated Contracting States:
**AT BE DE DK ES FI FR GB GR IE IT NL PT SE**

(30) Priority: **10.04.1996 US 15130 P**

(43) Date of publication of application:
**17.11.1999 Bulletin 1999/46**

(73) Proprietor: **Stoller Enterprises, Inc.**
**Houston, TX 77024 (US)**

(72) Inventor: **DEAN, Frank, William**
**Spring, TX 77373 (US)**

(74) Representative: **Ruschke, Hans Edvard, Dipl.-Ing.**
**Ruschke Hartmann Becker**
**Pienzenauerstrasse 2**
**81679 München (DE)**

(56) References cited:

**DD-A- 90 137** **US-A- 4 315 763**
**US-A- 4 935 413** **US-A- 4 950 323**

- **BEILSTEIN INFORMATION SERVICE FILE: XFIRE, XP002155215**
- **COLLECT. CZECH. CHEM. COMMUN., 1992, Vol. 57, JIRMAN J. et al., "170 NMR Spectra of Acylated Ureas and Thioureas", pages 1278-1281, XP000944775**
- **COLLECT. CZECH. CHEM. COMMUN., 1987, Vol. 52, JIRMAN J., "15N, 13C and 1H NMR Spectra of Acylated Ureas and Thioureas", pages 2474-2481, XP000944778**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 955 797 B1

## Description

BACKGROUND OF THE INVENTION

1. Field of the Invention

The present invention to the preparation of new substituted ureas and to agricultural uses of those substituted ureas to improve plant growth. More specifically, the present invention is directed to a method for reacting a carboxylic acid with urea to form a new N,N'-di-substituted urea, in particular diformylurea, to a composition containing N,N'-diformylurea as an active substance and to the use of this composition in a method for enhancing the growth of a plant as well as to a treated plant seed to which N,N'-diformylurea has been applied and to the diformylurea per se.

2. Description of the Background

Urea, being approximately 46% by weight nitrogen, has long been preferred as a nitrogen source for fertilizing soils to stimulate plant growth.

However, urea suffers from high ammonia losses when used in the presence of moisture. This disadvantage effectively restricted the use of urea for many years. It is believed that these losses are caused by the hydrolysis of urea in the presence of moisture and the enzyme urease. The addition of a water soluble salt to aqueous solutions of urea has been suggested as a means for reducing ammonia volatilization (see US-A-4,500,335).

While substituted ureas are also known, e.g., diphenylurea, they have found little agricultural use. Urea undergoes condensation reactions with carboxylic acids to produce barbituates and their analogs. However, these products have found no agricultural uses.

High analyses liquid fertilizers comprising the reaction product of urea and phosphoric acid are known from US-A-4,315,763. However, diformylurea or any other N,N'-di-substituted ureas are not disclosed therein. This also applies to DD patent 90, 137 which relates to a process for preparing acetylurea by reacting urea with acetic acid in the presence of Lewis acids as catalyst (see also Beilstein Information Service, XP-0021 55 215). Herbicidal bis-formyl-N-aryl-N'-alkyl ureas are known from US-A-4,950,323. These products are only used for killing plants and not for enhancing the growth of plants.

However, the argicutural industry has felt the need for ways to improve early seedling vigor and to increase plant biomass, both resulting in improved yield. There has been a long felt but unfulfilled need in the industry for improved methods for achieving these goals. The present invention solves those needs.

SUMMARY OF THE INVENTION

The present invention is directed to a method for preparing substituted ureas, in particular diformylurea, and to their use in a composition and in a method for enhancing the growth of a plant.

Subject-matter of the present invention is according to a first aspect a method for preparing a substituted urea having the formula

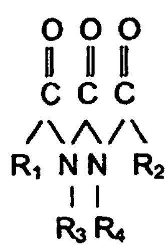

where $R_1$, $R_2$, $R_3$ and $R_4$ are the same or different and are selected from the group consisting of hydrogen, substituted and unsubstituted alkyl, allyl, vinyl and alkoxyl groups having from 1 to 6 carbon atoms, substituted and unsubstituted phenyl groups and the halides,
the method comprising reacting a carboxylic acid with a reactant selected from the group consisting of urea, N-mono-substituted ureas and N,N'-di-substituted ureas.

The method of the present invention preferably comprises each of the following preferred features, each taken alone or in any combination:

the carboxylic acid has the formula RCOOH, where R is selected from the group consisting of hydrogen, substituted and unsubstituted alkyl, allyl, vinyl and alkoxyl groups having from 1 to 6 carbon atoms, substituted and unsubstituted phenyl groups and the halides;

the reactant has the formula $(NHR')_2CO$, where each R' is the same or different and is selected from the group consisting of hydrogen, substituted and unsubstituted alkyl groups having from 1 to 6 carbon atoms, substituted and unsubstituted alkoxyl groups having from 1 to 6 carbon atoms, substituted and unsubstituted phenyl groups and the halides;

the used carboxylic acid is preferably formic acid or acetic acid;

the used reactant is preferably urea;

the molar ratio of carboxylic acid to the reactant is about 2 : 1;

the carboxylic acid and the reactant are reacted at a temperature of from 10 to 140 °C, preferably of from 15 to 40 °C;

$R_3$ and $R_4$ in the substituted urea are hydrogen;

$R_1$ and $R_2$ in the substituted urea are selected from the group consisting of hydrogen and alkyl groups having from 1 to 3 carbon atoms;

$R_1$ and $R_2$ in the substituted urea are hydrogen;

the carboxylic acid is preferably formic acid and the reactant is preferably urea; and

the carboxylic acid and the reactant are reacted at about room temperature and with stirring until clear.

Subject-matter of the present invention is according to a second aspect a composition for enhancing the growth of a plant comprising as an active substance N,N'-diformylurea produced by the methods as defined above and having the formula

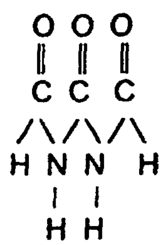

According to a third aspect the present invention relates to a use of the composition as defined above in a method for enhancing the growth of a plant wherein the composition is applied to seeds for said plant prior to planting, to soil surrounding said plant or to foliage of said plant.

The composition preferably is used in the form of an aqueous solution having a concentration of from 0.001 to 1.0 M of said N,N'-diformylurea which is applied to said seeds at a rate of from 15 to 750 ml of solution per 454 kg of seeds;
wherein said seeds are soaked in said solution for 2 to 48 hours prior to planting.

The composition is preferably used in the form of an aqueous solution having a concentration of from 0.0001 to 1.0 M of said N,N'-diformylurea and is applied to said foliage at a rate of from 1 to 100 g of said substituted urea per 4047 $m^2$ (acre).

Preferably said solution further comprises a vegetable oil and a surfactant;
said vegetable oil being preferably selected from the group consisting of sunflower oil and soybean oil and said surfactant being related from the group consisting of organic polyphosphates and ethoxylated nonaphenols.

According to a fourth aspect the present invention relates to a treated plant seed to which N,N'-diformylurea having the following formula has been applied

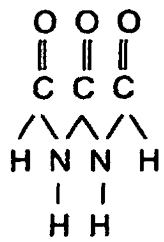

Preferably the treated seed has been soaked in an aqueous solution containing said N,N'-diformylurea.

According to a fifth aspect the present invention relates to diformylurea per se having the formula

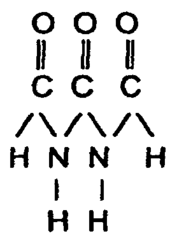

The reaction product of the method of the present invention comprises an N,N'-di-substituted urea having the formula

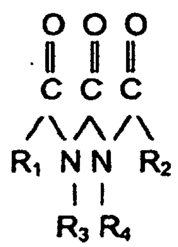

where $R_1$, $R_2$, $R_3$ and $R_4$ are the same or different and are selected from the group consisting of hydrogen, substituted and unsubstituted alkyl, allyl, vinyl and alkoxyl groups having from one to six carbon atoms substituted and unsubstituted phenyl groups and the halides.

In a preferred embodiment, the second reactant is unsubstituted urea so that $R_3$ and $R_4$ in the reaction product are hydrogen. In a more preferred embodiment, $R_1$ and $R_2$ are selected from the group consisting of hydrogen and alkyl groups having from one to three carbon atoms. Most preferably, formic acid is reacted with urea in a molar ratio of about 2:1 to produce N,N'-diformylurea.

While the reaction may be conducted at any temperature between 10 °C and 140 °C, it is preferably conducted within the range of 15 °C to 40 °C. The reaction may conveniently be conducted at room temperature. Preferably the reactants are stirred until the reaction mixture is clear. Crystals of the reaction product will form and may be separated from the reaction mixture.

The reaction products of the method of the present invention, most preferably N,N'-diformylurea, have been found to produce enhanced growth in plants when used in a variety of ways. These reaction products, most preferably diformylurea, produce enhanced growth when applied to seeds prior to planting, when applied to the soil surrounding the plant at or after planting or when applied to the foliage of the plant, e.g., at the three leaf stage of growth.

An aqueous solution containing from about 0.001-1.0 M of the reaction product may be applied to the surface of seeds at a rate of about 15-750 ml. of solution per 454 kg (100 lbs) of seed. In a convenient method, the solution may be applied to the surface of the seeds or the seeds may be soaked in such a solution for about 2-24 hours, preferably for about 24 hours, prior to planting. Alternatively, such a solution may be applied to the soil surrounding the seed and/or emerging plant. When so applied, the solution may be applied at a rate of about 1-100 grams of diformylurea or other reaction product per 4047 $m^2$ (acre).

Still another alternative is the application of such an aqueous solution to the foliage of the plant, preferably

at the three leaf growth stage, at a rate of about 1-100 grams of diformylurea or other reaction product per 4047 $m^2$ (acre). When applied to the foliage, those skilled in the art may include a conventional vegetable oil and surfactant in the solution to improve the retention of reaction product on the leaves so that it may be more readily absorbed by the plant.

The reaction products of urea and carboxylic acids described herein, particularly N,N'- diformylurea, have produced significantly improved growth when applied to the seeds and foliage of a variety of agricultural products. Accordingly, a significant improvement in crop development may be obtained using these new products. These and other meritorious features and advantages of the present invention will be more fully appreciated from the following detailed description and claims.

DETAILED DESCRIPTION OF THE PREFERRED EMBODIMENT

The present invention provides methods for preparing and using the reaction products of a carboxylic acid and a diformylurea comprising new composition of matter. It has been found that these reaction products may be easily prepared and have significant agricultural uses because of their perceived biological activity. In fact, it has been found that these reaction products, specifically N,N'-diformylurea, enhance the growth of a variety of agricultural crops when applied to the seeds, surrounding soil or foliage.

The reaction products of the method of the present invention have the general formula

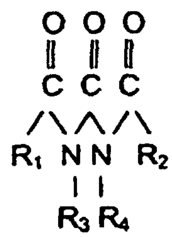

where $R_1$, $R_2$, $R_3$ and $R_4$ are the same or different and are selected from the group consisting of hydrogen, substituted and unsubstituted alkyl, allyl, vinyl and alkoxyl groups having from 16 carbon atoms, substituted and unsubstituted phenyl groups and the halides.

These reaction products are prepared by reacting a carboxylic acid having the formula RCOOH where R is selected from the group consisting of hydrogen, substituted and unsubstituted alkyl, allyl, vinyl and alkoxyl groups having from 1 to 6 carbon atoms, substituted and unsubstituted phenyl groups and the halides.

Exemplary acids include formic acid, acetic acid, propionic acid, butyric acid, valeric acid, caproic acid, heptanoic acid, and citric acid. Preferably R is selected from the group consisting of hydrogen and unsubstituted alkyl groups having from 1 to 3 carbon atoms. The presently most preferred acids are formic and acetic acid. These carboxylic acids are reacted with a substituted or unsubstituted urea having the formula $(NHR')_2$ CO where each R' is the same or different and is selected from the group consisting of hydrogen, substituted and unsubstituted alkyl groups having from 1 to 6 carbon atoms, substituted and unsubstituted alkoxyl groups having from 1 to 6 carbon atoms, substituted and unsubstituted phenyl groups and the halides. Unsubstituted urea is the presently most preferred reactant.

In its most preferred embodiment, the present invention comprises the reaction product of urea and formic acid, i.e., N,N'- diformylurea, having the following formula

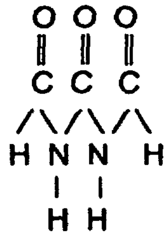

It has been found that the reaction of the present invention will proceed throughout a wide range of temperatures, e.g., from 10 °C to 140 °C, restricted only by the boiling points of the reactants and products. While heat may

be added by any conventional means to speed the rate of these reactions, it has been found that the methods of the present invention may conveniently be performed in a temperature range of from 15 °C to 40 °C, preferably at room temperature, i.e., from 20 °C to 30 °C. These reactions appear to be slightly exothermic. The reaction of formic acid and urea to form diformylurea proceeds to completion within 24 hours at room temperature. It is preferred that the reaction mixture be stirred until clear and then permitted to remain quiescent until crystals of the reaction product have formed.

It is believed that the reactions proceed by the elimination of two water molecules. The reaction of urea and formic acid proceeds as follows:

$$H_2NCONH_2 + 2RCOOH \rightarrow RCONHCONHCOR + 2\ H_2O$$

In this reaction, formic acid reacts with one hydrogen on each of the urea nitrogens to produce N,N'-diformylurea. Accordingly, it is preferred that the reaction mixture comprises about 2 moles of carboxylic acid for each mole of urea.

These reaction products are biologically active as a result of the similarity of their skeletal structure, i.e., the nitrogen-carbon-oxygen skeleton, with the alternating double bond structure of these same elements in a variety of synthetic and naturally occurring biological molecules.

Thus, these reaction products, e.g., N, N'- diformylurea, will find a variety of biological uses. These compounds may be used to produce, not only the improved plant growth shown herein, but with the substitution of appropriate functional groups or bulky substituents, a variety of effective algaecides, herbicides, fungicides or pesticides may be produced.

Applicant believes that the above reaction products, particularly N,N'-diformylurea, may mimic plant growth hormones and/or plant growth regulators based upon the similarity of their skeletal structure to a variety of biologically active compounds. Common to all biologically active molecules in this class is a core structure including both alternating double bonds and alternating carbon to nitrogen bonds. These structures are common in all synthetically produced and naturally occurring biologically active molecules, e.g., cytokinins, substituted uracils, methylguanine and the like. While adenine and guanine have a fused ring structure, cytosine, thymine and uracil exhibit the same structures as pyrimidines. Because the N,N'- di-substituted ureas prepared according to the present invention, e.g., diformylurea, are linear, they can conform to the shapes of these biological molecules. While this conformation is not exact, it is believed that this feature will facilitate the biological activity of these molecules.

The reaction products of the method of the present invention, specifically N,N'-diformylurea, have been used to enhance the growth of plants. In fact, it has been found that improved growth may be obtained by applying diformylurea to the seeds, or to the soil surrounding the plant, or to the foliage of the plant. A single application of diformylurea has been found to produce significantly greater growth in a variety of crops, including wheat, corn, peanuts, soybeans, rice and cotton. For example, a single application to rice at the rate of 50 grams per 4047 (acre) at tillering has produced a 25 percent increase in yield.

In one method of the present invention, seeds are treated with an aqueous solution containing the N,N'-disubstituted reaction product of a carboxylic acid and urea. Seeds may conveniently be soaked in an aqueous solution of the reaction product for a time from about 2 to 24 hours. Excellent results have been obtained with seeds soaked in an aqueous solution of diformylurea for about 24 hours. The seeds may be immediately planted or may be dried to produce a seed which has been treated with the reaction product.

While those skilled in the art will be able to prepare aqueous solutions of the desired concentration for these agricultural uses, it has been found that solutions containing from about 0.001 to 1.0 M of the reaction product are typically appropriate. Aqueous solutions containing from about 0.001 to 0.050 M are presently preferred. While these solutions may be applied at any rate desired by those of skill in the art, it has been found that aqueous solutions of the foregoing concentration provide good results when applied at the rate of about 15-750 ml per 454 kg (100 lbs) of seed. Alternatively, it is believed that the reaction products of the method of the present invention, typically in aqueous solutions of the foregoing concentrations, may be added to the soil surrounding the seed at planting or after emergence of the plant.

In another alternative method, excellent results have been obtained by a one time spraying of the foliage of the emerging plant, preferably at the three leaf stage, with an aqueous solution containing the reaction product. Those skilled in the art would be aware that addition of a small quantity of oil and/or surfactant to the aqueous solution sprayed on the foliage will improve the adherence of the reaction product to the leaves and the uptake of the reaction product by the plant. Suitable oils include both saturated and unsaturated oils, alcohols, esters and other compounds having both hydrosphobic and hydrophilic functional groups. Exemplary oils comprise the vegetable oils and include sunflower oil and soybean oil. Exemplary biologically acceptable surfactants include the organic polyphosphates and ethoxylated nonophenols. Again, those skilled in the art can determine appropriate concentrations for each desired use.

However, aqueous solutions having the foregoing concentrations are believed to be generally appropriate. These solutions should be applied at a rate sufficient to provide about 1-100 grams of reaction product per 4047 $m^2$ (acre).

The following are examples illustrating methods for preparing reaction products of the present invention, specifically N,N'- diformylurea and N,N'diacetylurea, and results of applying diformylurea to a variety of agricultural crops.

Preparation of N,N'-Diformylurea

Diformylurea has been prepared by reacting formic acid with urea in a molar ratio of about 1.8-2.0 moles formic acid to 1.0 mole urea. In a first example, 600 grams of urea was reacted with 920 grams of 90% formic acid.

The molar ratio of formic acid to urea in this example is about 1.8 to 1. In a second example, 555 grams of urea was reacted with 945 grams of 90% formic acid. In this example, the molar ratio of formic acid to urea is about 2:1. The reaction mixture of both examples was stirred for about 1 hour until the solution was clear. The clear solution was allowed to stand for about 48 hours at room temperature. Within 24 hours, substantially all of the reaction mixture had crystalized. At about 48 hours, the remaining liquid was decanted and the crystals purified by rinsing with the mother liquor and ice water using vacuum filtration. Upon analysis, only a single reaction product, i.e., N,N'- diformylurea, was found. Unexpectedly the formic acid had reacted on both of the urea nitrogens.

Preparation of N,N'-Diacetylurea

Diacetylurea was prepared by reacting 219 grams of urea with 438 grams of 99% acetic acid. The molar ratio of acetic acid to urea is about 2:1. The reaction mixture was stirred for about 1 hour until clear as before. Upon standing, crystals of N,N'-diacetylurea formed. The crystals were purified as described above.

Agricultural Use of N,N'-Diformylurea

In a preliminary experiment to determine the agricultural value of the N,N'-diformylurea prepared above, a concentrate was prepared by mixing 200 grams diformylurea and 200 grams formic acid, adjusting the ph to about 5.5 by the addition of potassium hydroxide and adding sufficient water to make 1 liter. This results in a concentrate having a concentration of 1.7 M diformylurea and 4.3 M formate. Low, medium and high dilution aqueous solutions were prepared by diluting, respectively, 0.8 grams, 8.0 grams and 40.0 grams of the concentrate per liter of final solution. Thus, the concentration of diformylurea in the low dilution was 0.0013 M. The concentration of diformylurea in the medium dilution was 0.013 M, while the high concentration dilution had a concentration of 0.068 M diformylurea.

The foregoing solutions were used to coat corn, soybean, cotton and rice seeds prior to planting. Coating was achieved by soaking the seeds in the foregoing solutions for about 24 hours. Sufficient solution was used to cover the seeds. Control seeds were soaked in water for the same time.

Untreated or control seeds, together with seeds treated by the low, medium and high dilution solutions, were planted in 3.8 l (1 gallon) greenhouse pots.

Six pots were used for each dilution or control with five seeds planted per pot.

The plants were thinned to two plants per pot after emergence. The plants were watered and fertilized as required. After 30 days the plants were harvested and the roots and shoots examined.

The roots and shoots were weighed separately and the root/shoot ratio determined. The treated plants were generally characterized by both greater root mass and shoot mass when compared to the controls. The root/shoot ratio for most of the treated plants was greater than that for the controls. Further, the older leaves of plants grown from the treated seeds appeared to exhibit greater length and width than those from plants grown from the untreated seeds. Finally, crown roots were noticed on both corn and rice plants grown from the treated seeds. The results of these preliminary tests are summarized in Table I.

Table I

| | | Seed Treated | | | |
|---|---|---|---|---|---|
| | | Control | Low | Medium | High |
| Cotton | Roots (gm) | 3.03 | 7.85 | 4.50 | 5.07 |
| | Shoots (gm) | 4.57 | 5.38 | 5.25 | 6.90 |
| | Total (qm) | 7.60 | 13.23 | 9.75 | 11.97 |
| | Root/Shoot Ratio | 0.66 | 1.46 | 1.08 | 0.73 |

Table I (continued)

| | | Seed Treated | | | |
|---|---|---|---|---|---|
| | | Control | Low | Medium | High |
| | | | | | |
| Rice | Roots (gm) | 0.47 | 0.61 | 3.07 | 0.82 |
| | Shoots (gm) | 0.24 | 0.34 | 1.84 | 0.39 |
| | Total (gm) | 0.71 | 0.95 | 4.91 | 1.21 |
| | Root/Shoot Ratio | 1.95 | 1.78 | 1.86 | 2.10 |
| | | | | | |
| Corn | Roots (gm) | 9.00 | 9.80 | 10.40 | 9.60 |
| | Shoots (gm) | 9.60 | 9.40 | 9.60 | 8.80 |
| | Total (gm) | 18.60 | 19.20 | 20.00 | 18.40 |
| | Root/Shoot Ratio | 0.94 | 1.04 | 1.08 | 1.09 |
| | | | | | |
| Soybean | Roots (gm) | 2.40 | 4.60 | 4.90 | - |
| | Shoots (gm) | 3.80 | 4.90 | 4.10 | - |
| | Total (gm) | 6.20 | 9.50 | 9.00 | - |
| | Root/Shoot Ratio | 0.63 | 0.93 | 1.20 | - |

Table I illustrates the improved growth achieved by treating seeds prior to planting with an aqueous solution containing diformylurea. In substantially every example the treated seeds produced plants having greater root mass, greater shoot mass and greater total mass. Further, the ratio of root to shoot mass was increased in all of the treated cotton, corn and soybean samples.

Based upon the foregoing preliminary work, additional experiments to investigate the effect of diformylurea on other plant species was undertaken.

These experiments were also designed to compare the effect of treating seeds as described above with foliar treatment. Wheat, corn and peanuts were selected for the next phase of this work. Diformylurea was prepared by reacting formic acid and urea at a molar ratio of 2:1 as described above. An aqueous solution containing diformylurea at a concentration of 0.01 M was prepared. For the seed treatment portion of the experiments, the seeds to be treated were soaked in the foregoing solution for about 24 hours before planting. The control seeds were again soaked in water for the same time. Both control and treated seeds were planted in 1 gallon greenhouse pots. Five seeds were planted in each pot. Each pot was thinned to two plants after germination. For the foliar treatment portion of the experiment, plants grown from untreated seeds were sprayed with a solution containing 0.01 M. diformylurea at the three leaf stage.

All plants were watered and fertilized as required. Thirty days after planting, the plants were harvested and the roots and shoots examined. The mass of both the roots and shoots and the total mass of the plant in each pot were determined. The results for these experiments for control, seed treated and foliar treated wheat, corn and peanuts are reported in Tables II-IV, respectively.

## Table II

| Sample | Wheat | | | | | | | | |
|---|---|---|---|---|---|---|---|---|---|
| | Control | | | Seed Treated 0.01 M diformylurea | | | Foliar Treated 0.01 M diformylurea | | |
| | Roots (gm) | Shoots (gm) | Total (gm) | Roots (gm) | Shoots (gm) | Total (gm) | Roots (gm) | Shoot s (gm) | Total (gm) |
| 1 | 12.10 | 1.41 | 13.51 | 46.50 | 9.76 | 56.26 | 14.00 | 4.00 | 18.00 |
| 2 | 30.04 | 3.19 | 33.23 | 65.06 | 12.94 | 78.00 | 26.81 | 7.28 | 34.09 |
| 3 | 29.63 | 5.15 | 34.78 | 27.98 | 4.17 | 32.15 | 37.26 | 7.48 | 44.74 |
| 4 | 14.98 | 2.03 | 17.01 | 41.69 | 12.22 | 53.91 | 25.13 | 7.53 | 32.66 |
| 5 | | | | 59.01 | 15.66 | 74.67 | 20.12 | 4.44 | 24.56 |
| 6 | 17.90 | 2.36 | 20.26 | 32.14 | 7.32 | 39.46 | 20.28 | 4.62 | 24.90 |
| average | 20.93 | 2.83 | 23.76 | 45.40 | 10.35 | 55.74 | 23.93 | 5.89 | 29.83 |

**Table III**

| Sample | Corn | | | | | | | | |
|---|---|---|---|---|---|---|---|---|---|
| | Control | | | Seed Treated 0.01 M diformylurea | | | Foliar Treated 0.01 M diformylurea | | |
| | Roots (gm) | Shoots (gm) | Total (gm) | Roots (gm) | Shoots (gm) | Total (gm) | Roots (gm) | Shoot s (gm) | Total (gm) |
| 1 | 55.31 | 43.19 | 98.50 | 57.78 | 46.12 | 103.90 | 42.18 | 67.57 | 109.75 |
| 2 | 50.53 | 26.16 | 76.69 | 80.82 | 34.73 | 115.55 | 66.57 | 56.61 | 123.18 |
| 3 | 50.91 | 24.55 | 75.46 | 38.15 | 17.33 | 55.48 | 42.40 | 61.80 | 104.20 |
| 4 | 30.95 | 29.65 | 60.60 | 57.78 | 46.12 | 103.90 | 48.44 | 46.17 | 84.61 |
| 5 | 39.73 | 17.53 | 57.26 | 80.82 | 34.73 | 115.55 | 52.48 | 50.59 | 103.07 |
| 6 | 45.49 | 28.22 | 73.71 | 38.15 | 17.33 | 55.48 | 56.22 | 61.12 | 117.34 |
| average | 46.15 | 28.22 | 74.37 | 58.92 | 32.73 | 91.65 | 51.38 | 57.31 | 108.69 |

**Table IV**

| Sample | Peanuts | | | | | | | | |
|---|---|---|---|---|---|---|---|---|---|
| | Control | | | Seed Treated 0.01 M diformylurea | | | Foliar Treated 0.01 M diformylurea | | |
| | Roots (gm) | Shoots (gm) | Total (gm) | Roots (gm) | Shoots (gm) | Total (gm) | Roots (gm) | Shoots (gm) | Total (gm) |
| 1 | 18.46 | 14.66 | 33.12 | 16.46 | 20.20 | 36.66 | 31.69 | 36.17 | 67.86 |
| 2 | 17.29 | 10.86 | 28.15 | 22.65 | 22.00 | 44.65 | 33.55 | 31.82 | 65.37 |
| 3 | 15.96 | 10.60 | 26.56 | 20.11 | 20.46 | 40.57 | 27.67 | 28.38 | 56.05 |
| 4 | 13.19 | 14.77 | 27.96 | 22.04 | 22.34 | 44.38 | 35.04 | 30.34 | 65.38 |
| 5 | 19.52 | 17.85 | 37.37 | 21.60 | 19.03 | 40.63 | 37.38 | 33.84 | 71.22 |
| 6 | 17.92 | 23.90 | 41.82 | 16.74 | 17.77 | 34.51 | 31.10 | 32.35 | 63.45 |
| average | 17.06 | 15.44 | 32.50 | 19.93 | 20.30 | 40.23 | 32.74 | 32.15 | 64.89 |

A review of Tables II-IV establishes that the root mass, shoot mass and total plant mass appear to be increased in response to both treatment of the seeds and foliar treatment of the emerging leaves with an aqueous solution of diformylurea for each of the three tested crops. Average weights for the plants grown from untreated, seed treated and foliar treated seeds for each crop are reported in Table V.

**Table V**

| | | Wheat | | | Corn | | | Peanuts | | |
|---|---|---|---|---|---|---|---|---|---|---|
| | | Control | Seed Treated | Foliar Treated | Control | Seed Treated | Foliar Treated | Control | Seed Treated | Foliar Treated |
| | Roots (gm) | 20.93 | 45.40 | 23.93 | 46.15 | 58.92 | 51.38 | 17.06 | 19.93 | 32.74 |
| | Shoots (gm) | 2.83 | 10.35 | 5.89 | 28.22 | 32.73 | 57.31 | 15.44 | 20.30 | 32.15 |
| | Total (gm) | 23.76 | 55.74 | 29.83 | 74.37 | 91.65 | 108.69 | 32.50 | 40.23 | 64.89 |
| | Root/Shoot Ratio | 7.40 | 4.39 | 4.06 | 1.61 | 1.80 | 0.90 | 1.10 | 0.98 | 1.02 |
| Percent Change v. Control | Roots | | 117 | 14 | | 30 | 11 | | 17 | 92 |
| | Shoots | | 268 | 108 | | 16 | 103 | | 31 | 108 |
| | Total | | 135 | 26 | | 24 | 46 | | 24 | 100 |

The observed average weights for the roots, shoots and total mass, together with the percentage change

versus control, for each of the three crops for both seed and foliar treatment are listed in Table V. Root mass, shoot mass and total mass increased for each of these three crops when treated with diformylurea, whether seed or foliar applied. The mass of the seed treated wheat was 135% greater than the control while that of the foliar treated peanuts was 100% greater. The remaining total mass results showed increases of 24-46 % with respect to the control. The root to shoot ratios in these experiments were mixed. It appears that increased plant mass, both root and shoot, will be achieved by application of diformylurea to the seeds prior to planting or to the foliage after emergence.

Additional experiments were designed to study further the effect of the concentration of diformylurea applied to the seeds of several other crops. These experiments were conducted using cotton, rice, corn and soybeans. These experiments were similar to the preliminary experiments described above. The concentrate described above, containing both diformylurea and formate, was used to prepare solutions containing low, medium and high concentrations of diformylurea having, respectively, 0.001 M, 0.01 M and 0.05 M diformylurea. These solutions were used to treat the seeds of cotton, rice, corn and soybeans.

Seeds of each of these crops were soaked in water or the prepared aqueous solutions of diformylurea for 24 hours before planting. Five seeds were planted in each of six, 3,8 l (1 gallon) greenhouse pots. Pots were thinned to two plants after germination and the plants watered and fertilized as needed for 30 days.

Thereafter, the plants were harvested and the mass of the roots, shoots and total mass determined. Those results, together with the averages are reported in Tables VI-IX.

## Table VI

| | Cotton | | | | | | | | | | | |
|---|---|---|---|---|---|---|---|---|---|---|---|---|
| | Control | | | Seed Treated 0.001M diformylurea | | | Seed Treated 0.01 M diformylurea | | | Seed Treated 0.05 M diformylurea | | |
| Sample | Roots (gm) | Shoots (gm) | Total (gm) | Roots (gm) | Shoots (gm) | Total (gm) | Roots (gm) | Shoots (gm) | Total (gm) | Roots (gm) | Shoots (gm) | Total (gm) |
| 1 | 4.70 | 4.80 | 9.50 | 6.90 | 5.70 | 12.60 | 6.90 | 4.80 | 11.70 | 4.70 | 6.90 | 11.60 |
| 2 | 1.50 | 4.50 | 6.00 | 9.20 | 5.70 | 14.90 | 2.40 | 4.60 | 7.00 | 6.60 | 7.00 | 13.60 |
| 3 | 2.90 | 4.40 | 7.30 | 3.80 | 4.80 | 8.60 | 1.90 | 4.00 | 5.90 | 4.30 | 4.70 | 9.00 |
| 4 | | | | 8.00 | 4.40 | 12.40 | 4.20 | 6.00 | 10.20 | 6.00 | 5.00 | 11.00 |
| 5 | | | | 10.00 | 6.20 | 16.20 | 7.60 | 4.90 | 12.50 | 4.20 | 6.40 | 10.60 |
| 6 | | | | 9.20 | 5.50 | 14.70 | 4.00 | 7.20 | 11.20 | 4.60 | 5.40 | 10.80 |
| Average | 3.03 | 4.57 | 7.60 | 7.85 | 5.38 | 13.23 | 4.50 | 5.25 | 9.75 | 5.07 | 5.90 | 10.97 |

## Table VII

| | Rice | | | | | | | | | | | |
|---|---|---|---|---|---|---|---|---|---|---|---|---|
| | Control | | | Seed Treated 0.001M diformylurea | | | Seed Treated 0.01 M diformylurea | | | Seed Treated 0.05 M diformylurea | | |
| Sample | Roots (gm) | Shoots (gm) | Total (gm) | Roots (gm) | Shoots (gm) | Total (gm) | Roots (gm) | Shoots (gm) | Total (gm) | Roots (gm) | Shoots (gm) | Total (gm) |
| 1 | 0.58 | 0.26 | 0.85 | 0.93 | 0.46 | 1.39 | 0.40 | 0.23 | 0.63 | 0.38 | 0.30 | 0.68 |
| 2 | 0.34 | 0.17 | 0.51 | 0.59 | 0.34 | 0.93 | 0.78 | 0.40 | 1.18 | 0.94 | 0.33 | 1.27 |
| 3 | 0.42 | 0.29 | 0.71 | 0.20 | 0.13 | 0.33 | 0.52 | 0.26 | 0.78 | 1.19 | 0.46 | 1.65 |
| 4 | | | | 0.55 | 0.37 | 0.92 | 0.27 | 0.13 | 0.40 | 0.21 | 0.18 | 0.39 |
| 5 | | | | 1.11 | 0.52 | 1.62 | 0.45 | 0.38 | 0.83 | 0.78 | 0.41 | 1.19 |
| 6 | | | | 0.28 | 0.23 | 0.51 | 0.65 | 0.44 | 1.09 | 1.43 | 0.63 | 2.06 |
| Average | 0.45 | 0.24 | 0.69 | 0.61 | 0.34 | 0.95 | 0.51 | 0.31 | 0.82 | 0.82 | 0.39 | 1.21 |

## Table VIII

| | Corn | | | | | | | | | | | |
|---|---|---|---|---|---|---|---|---|---|---|---|---|
| | Control | | | Seed Treated 0.001M diformylurea | | | Seed Treated 0.01 M diformylurea | | | Seed Treated 0.05 M diformylurea | | |
| Sample | Roots (gm) | Shoots (gm) | Total (gm) | Roots (gm) | Shoots (gm) | Total (gm) | Roots (gm) | Shoots (gm) | Total (gm) | Roots (gm) | Shoots (gm) | Total (gm) |
| 1 | 20.50 | 18.80 | 39.30 | 18.10 | 18.90 | 37.00 | 15.30 | 15.50 | 30.80 | 21.20 | 18.40 | 39.60 |
| 2 | 18.30 | 17.90 | 36.20 | 17.60 | 19.50 | 37.10 | 24.80 | 19.70 | 44.50 | 18.70 | 15.80 | 34.50 |
| 3 | 19.00 | 17.10 | 36.10 | 18.90 | 21.10 | 40.00 | 16.20 | 18.60 | 34.80 | 17.90 | 17.40 | 35.30 |
| 4 | | | | 17.20 | 14.00 | 31.20 | 25.10 | 22.10 | 47.20 | 20.40 | 20.60 | 41.00 |
| 5 | | | | 27.00 | 17.60 | 44.60 | 24.10 | 23.00 | 47.10 | 17.80 | 15.90 | 33.70 |
| 6 | | | | 19.10 | 22.00 | 41.10 | 20.00 | 16.60 | 36.60 | | | |
| Average | 19.27 | 17.93 | 37.20 | 19.65 | 18.85 | 38.50 | 20.92 | 19.17 | 40.09 | 19.20 | 17.82 | 38.82 |

**Table IX**

| | Soybean | | | | | | | | |
|---|---|---|---|---|---|---|---|---|---|
| | Control | | | Seed Treated 0.001 M diformylurea | | | Seed Treated 0.01M diformylurea | | |
| | Roots (gm) | Shoots (gm) | Total (gm) | Roots (gm) | Shoots (gm) | Total (gm) | Roots (gm) | Shoots (gm) | Total (gm) |
| 1 | 5.10 | 7.90 | 13.00 | 7.80 | 8.70 | 16.50 | 12.40 | 9.90 | 22.30 |
| 2 | 4.50 | 6.90 | 11.40 | 8.40 | 9.80 | 18.20 | 5.70 | 8.90 | 14.60 |
| 3 | 5.00 | 8.10 | 13.10 | 7.30 | 10.20 | 17.50 | 8.40 | 11.20 | 19.60 |
| 4 | | | | 8.00 | 10.30 | 18.30 | 5.00 | 6.00 | 11.00 |
| 5 | | | | 11.30 | 9.30 | 20.60 | 5.90 | 8.30 | 14.20 |
| 6 | | | | 12.30 | 10.90 | 23.20 | | | |
| Average | 4.87 | 7.63 | 12.50 | 9.18 | 9.87 | 19.05 | 7.48 | 8.86 | 16.34 |

Review of the data reported in the foregoing tables illustrates that cotton, rice, corn and soybean seeds treated with an aqueous solution of diformylurea consistently produced plants having increased root, shoot and total plant mass.

These improved results were obtained over a wide concentration range from at least about 0.001 M diformylurea to at least about 0.05 M diformylurea in the aqueous solution in which the seeds were soaked. Table X illustrates the percentage change with respect to control for the root, shoot and total plant mass in these experiments.

Table X

| | | Percent Change v. Control | | |
|---|---|---|---|---|
| | Root/Shoot Ratio | Roots | Shoots | Total |
| Cotton | | | | |
| Control | 0.66 | - | - | - |

Table X (continued)

| | | Percent Change v. Control | | |
|---|---|---|---|---|
| | **Root/Shoot Ratio** | **Roots** | **Shoots** | **Total** |
| Cotton | | | | |
| 0.001M diformylurea | 1.46 | 159 | 18 | 74 |
| 0.01 M diformylurea | 0.86 | 49 | 15 | 28 |
| 0.05M diformylurea | 0.86 | 67 | 29 | 44 |
| | | | | |
| Rice | | | | |
| Control | 1.86 | - | - | - |
| 0.001M diformylurea | 1.79 | 37 | 42 | 39 |
| 0.01M diformylurea | 1.67 | 13 | 29 | 19 |
| 0.05M diformylurea | 2.13 | 82 | 63 | 76 |
| | | | | |
| Corn | | | | |
| Control | 1.07 | - | - | - |
| 0.001M diformylurea | 1.04 | 2 | 5 | 3 |
| 0.01 M diformylurea | 1.09 | 9 | 7 | 8 |
| 0.05M diformylurea | 1.09 | 0 | -2 | -1 |
| | | | | |
| Soybean | | | | |
| Control | 0.64 | - | - | - |
| 0.001M diformylurea | 0.93 | 89 | 29 | 52 |
| 0.01M diformylurea | 0.84 | 54 | 16 | 31 |

In substantially every case, both root and shoot mass increased for plants emerging from seeds treated with diformylurea. The root to shoot ratio for these plants is also illustrated in Table X. The ratio increased for both cotton and soybean and remained generally the same for rice and com.

The foregoing description of the invention has been directed in primary part to particularly preferred embodiments in accord with the requirements of the Patent Statute and for purposes of explanation and illustration. It will be apparent, however, to those skilled in the art that many modifications and changes in the specifically described system may be made without departing from the true scope and spirit of the invention. For example, while most of the work reported herein employs diformylurea, other N,N'- di-substituted ureas comprising the reaction product of carboxylic acids and urea may also be found to provide improved results. Further, those skilled in the art will be aware that the concentration of reaction product in aqueous solution may be adjusted as required based upon the nature of each crop or the application equipment. Therefore, the invention is not restricted to the preferred embodiments described and illustrated but covers all modifications which may fall within the scope of the following claims.

## Claims

1. A method for preparing a substituted urea having the formula

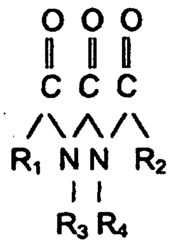

where $R_1$, $R_2$, $R_3$ and $R_4$ are the same or different and are selected from the group consisting of hydrogen, substituted and unsubstituted alkyl, allyl, vinyl and alkoxyl groups having from 1 to 6 carbon atoms, substituted and unsubstituted phenyl groups and the halides,
the method comprising reacting a carboxylic acid with a reactant selected from the group consisting of urea, N-mono-substituted ureas and N,N'-di-substituted ureas.

2. The method of claim 1, wherein the carboxylic acid has the formula RCOOH, where R is selected from the group consisting of hydrogen, substituted and unsubstituted alkyl, allyl, vinyl and alkoxyl groups having from 1 to 6 carbon atoms, substituted and unsubstituted phenyl groups and the halides.

3. The method of claim 1 or 2, wherein the reactant has the formula $(NHR')_2CO$, where each R' is the same or different and is selected from the group consisting of hydrogen, substituted and unsubstituted alkyl groups having from 1 to 6 carbon atoms, substituted and unsubstituted alkoxyl groups having from 1 to 6 carbon atoms, substituted and unsubstituted phenyl groups and the halides.

4. The method of any of claims 1 to 3, wherein said carboxylic acid is formic acid or acetic acid.

5. The method of any of claims 1 to 4, wherein said reactant is urea.

6. The method of any of claims 1 to 5 wherein the molar ratio of carboxylic acid to the reactant is about 2:1.

7. The method of any of claims 1 to 6 further comprising reacting said carboxylic acid and said reactant at a temperature of from 10 °C to 140 °C.

8. The method of claim 7 comprising reacting said carboxylic acid and said reactant at a temperature of from 15 °C to 40 °C.

9. The method of any of claims 1 to 8 wherein $R_3$ and $R_4$ are hydrogen.

10. The method of any of claims 1 to 9 wherein $R_1$ and $R_2$ are selected from the group consisting of hydrogen and alkyl groups having from 1 to 3 carbon atoms.

11. The method of any of claims 1 to 10 wherein $R_1$ and $R_2$ are hydrogen.

12. The method of any of claims 1 to 10 wherein the carboxylic acid is formic acid and the reactant is urea.

13. The method of any of claims 1 to 12 further comprising reacting said carboxylic acid and said reactant at about room temperature and with stirring until clear.

14. A composition for enhancing the growth of a plant comprising as an active substance N,N'-diformyl urea produced by the methods of any of claims 1 to 13 and having the formula

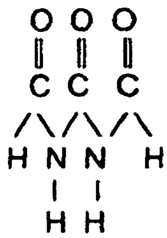

**15.** Use of the composition of claim 14 in a method for enhancing the growth of a plant wherein the composition is applied to seeds for said plant prior to planting, to soil surrounding said plant or to foliage of said plant.

**16.** The use of claim 15, wherein the composition in the form of an aqueous solution having a concentration of from 0.001 to 1.0 M of said N,N'-diformyl urea is applied to said seeds at a rate of from 15 to 750 ml of solution per 454 kg of seeds.

**17.** The use of claim 16 wherein said seeds are soaked in said solution for 2 to 48 hours prior to planting.

**18.** The use of claim 15 wherein the composition in the form of an aqueous solution having a concentration of from 0.0001 to 1.0 M of said N,N'-diformylurea is applied to said foliage at a rate of from 1 to 100 g of said substituted urea per 4047 $m^2$ (acre).

**19.** The use of any of claims 16 to 18, wherein said solution further comprises a vegetable oil and a surfactant.

**20.** The use of claim 19, wherein said vegetable oil is selected from the group consisting of sunflower oil and soybean oil and said surfactant is related from the group consisting of organic polyphosphates and ethoxylated nonaphenols.

**21.** A treated plant seed to which N,N'-diformylurea having the following formula has been applied

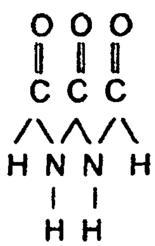

**22.** The treated seed of claim 21 which has been soaked in an aqueous solution containing said N,N'-diformylurea.

**23.** Diformylurea having the formula

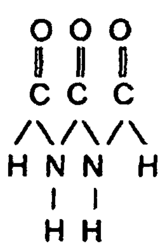

**Patentansprüche**

1. Verfahren zur Herstellung eines substituierten Harnstoffs der Formel

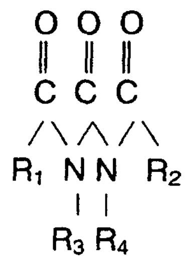

worin $R_1$, $R_2$, $R_3$ und $R_4$, die gleich oder verschieden sind, ausgewählt sind aus der Gruppe, die besteht aus Wasserstoff, substituierten und unsubstituierten Alkyl-, Allyl-, Vinyl- und Alkoxyl-Gruppen mit 1 bis 6 Kohlenstoffatomen, substituierten und unsubstituierten Phenyl-Gruppen und den Halogeniden,
das umfasst die Umsetzung einer Carbonsäure mit einem Reaktanten, ausgewählt aus der Gruppe, die besteht aus Harnstoff, N-mono-substituierten Harnstoffen und N,N'-di-substituierten Harnstoffen.

2. Verfahren nach Anspruch 1, worin die Carbonsäure die Formel RCOOH hat, worin R ausgewählt ist aus der Gruppe, die besteht aus Wasserstoff, substituierten und unsubstituierten Alkyl-, Allyl-, Vinyl- und Alkoxyl-Gruppen mit 1 bis 6 Kohlenstoffatomen, substituierten und unsubstituierten Phenyl-Gruppen und den Halogeniden.

3. Verfahren nach Anspruch 1 oder 2, worin der Reaktant die Formel $(NHR')_2CO$ hat, worin die jeweiligen Reste R', die gleich oder verschieden sind, ausgewählt sind aus der Gruppe, die besteht aus Wasserstoff, substituierten und unsubstituierten Alkylgruppen mit 1 bis 6 Kohlenstoffatomen, substituierten und unsubstituierten Alkoxyl-Gruppen mit 1 bis 6 Kohlenstoffatomen, substituierten und unsubstituierten Phenyl-Gruppen und den Halogeniden.

4. Verfahren nach einem der Ansprüche 1 bis 3, worin die Carbonsäure Ameisensäure oder Essigsäure ist.

5. Verfahren nach einem der Ansprüche 1 bis 4, worin der Reaktant Harnstoff ist.

6. Verfahren nach einem der Ansprüche 1 bis 5, worin das Molverhältnis von Carbonsäure zu Reaktant etwa 2:1 beträgt.

7. Verfahren nach einem der Ansprüche 1 bis 6, das außerdem umfasst die Umsetzung der Carbonsäure mit dem Reaktanten bei einer Temperatur von 10 bis 140 °C.

8. Verfahren nach Anspruch 7, das umfasst die Umsetzung der Carbonsäure mit dem Reaktanten bei einer Temperatur von 15 bis 40 °C.

9. Verfahren nach einem der Ansprüche 1 bis 8, worin $R_3$ und $R_4$ für Wasserstoff stehen.

10. Verfahren nach einem der Ansprüche 1 bis 9, worin $R_1$ und $R_2$ ausgewählt sind aus der Gruppe, die besteht aus Wasserstoff und Alkylgruppen mit 1 bis 3 Kohlenstoffatomen.

11. Verfahren nach einem der Ansprüche 1 bis 10, worin $R_1$ und $R_2$ für Wasserstoff stehen.

12. Verfahren nach einem der Ansprüche 1 bis 10, worin die Carbonsäure Ameisensäure ist und der Reaktant Harnstoff ist.

13. Verfahren nach einem der Ansprüche 1 bis 12, das außerdem umfasst die Umsetzung der Carbonsäure mit dem Reaktanten bei etwa Raumtemperatur und unter Rühren, bis die Reaktionsmischung klar ist.

14. Zusammensetzung zur Verbesserung des Wachstums einer Pflanze, die als aktive Substanz N,N'-Diformyl-Harnstoff, hergestellt nach dem Verfahren nach einem der Ansprüche 1 bis 13, der Formel umfasst:

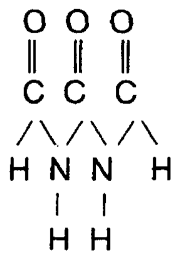

**15.** Verwendung der Zusammensetzung nach Anspruch 14 in einem Verfahren zur Verbesserung des Wachstums einer Pflanze, wobei die Zusammensetzung vor dem Einpflanzen auf Samen (Saatgut) für die Pflanze, auf die die Pflanze umgebende Erde oder auf das Laub der Pflanze aufgebracht wird.

**16.** Verwendung nach Anspruch 15, bei der die Zusammensetzung in Form einer wässrigen Lösung mit einer Konzentration von 0,001 bis 1,0 M N,N'-Diformylharnstoff auf die Samen (das Saatgut) in einer Menge von 15 bis 750 ml Lösung pro 454 kg Samen (Saatgut) aufgebracht wird.

**17.** Verwendung nach Anspruch 16, bei der die Samen (das Saatgut) vor dem Einpflanzen 2 bis 48 h lang in die Lösung eingetaucht werden (wird).

**18.** Verwendung nach Anspruch 15, bei der die Zusammensetzung in Form einer wässrigen Lösung mit einer Konzentration von 0,0001 bis 1,0 M N,N'-Diformylharnstoff auf das Laub in einer Menge von 1 bis 100 g des substituierten Harnstoffs pro 4047 $m^2$ aufgebracht wird.

**19.** Verwendung nach einem der Ansprüche 16 bis 18, bei der die Lösung außerdem ein Pflanzenöl und ein Tensid umfasst.

**20.** Verwendung nach Anspruch 19, bei der das Pflanzenöl ausgewählt wird aus der Gruppe, die besteht aus Sonnenblumenöl und Sojabohnenöl, und das Tensid stammt aus der Gruppe der organischen Polyphosphate und ethoxylierten Nonaphenole.

**21.** Behandelte Pflanzensamen, auf die N,N'-Diformylharnstoff mit der folgenden Formel aufgebracht worden ist

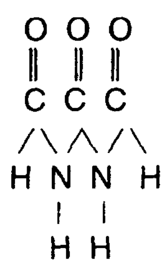

**22.** Behandelte Samen nach Anspruch 21, die in eine den N,N'-Diformylharnstoff enthaltende wässrige Lösung eingetaucht worden sind.

**23.** Diformylharnstoff der Formel

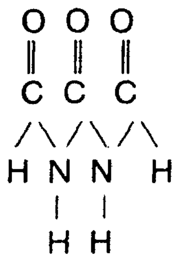

## Revendications

1. Procédé pour préparer une urée substituée ayant la formule

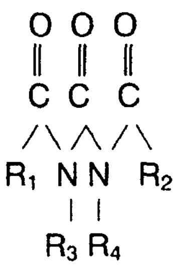

das laquelle $R_1$, $R_2$, $R_3$ et $R_4$, étant identiques ou différents, sont choisis dans un groupe consistant en l'hydrogène, des radicals alkyle, allyle, vinyle et alkoxyle contentenant 1 à 6 atomes de carbone et étant substitués ou non-substitués, des radicals phényle et des halogenures,
le procédé comprenant la réaction entre un acide carboxylique et un réactant choisi dans un groupe consistant en l'urée, des urées N-mono-substituées et des urées N,N'-di-substituées.

2. Procédé selon la revendication 1, dans lequel l'acide carboxylique a la formule RCOOH, das laquelle R est choisi dans un groupe consistant en l'hydrogène, des radicals alkyle, allyle, vinyle et alkoxyle contentenant 1 à 6 atomes de carbone et étant substitués ou non-substitués, des radicals phényle et des halogenures.

3. Procédé selon la revendications 1 ou 2, dans lequel le réactant a la formule $(NHR')_2CO$, dans laquelle les radicals R', étant identiques ou différents, sonst choisis dans un groupe consistant en l'hydrogène, des radicals alkyle contenants 1 à 6 atomes de carbone et étant substitués ou non-substitués, des radicals alkoxyle contenant, 1 à 6 atomes de carbone et étant substitués ou non-substitués, des radicals phényle substitués ou non-substitués et des halogenures.

4. Procédé selon l'une des revendications 1 à 3, dans lequel l'acide carboxylique est l'acide formique ou l'acide acétique.

5. Procédé selon l'une des revendications 1 à 4, dans lequel le réactant est l'urée.

6. Procédé selon l'une des revendications 1 à 5, dans lequel le rapport molaire de l'acide carboxylique sur le réactant est environ 2:1.

7. Procédé selon l'une des revendications 1 à 6 comprenant en plus la réaction entre l'acide carboxylique et le réactant à une température entre 10 et 140 °C.

8. Procédé selon la revendication 7 comprenant la réaction entre l'acide carboxylique et le réactant à une température

entre 15 et 40 °C.

**9.** Procédé selon l'une des revendications 1 à 8, dans lequel $R_3$ et $R_4$ représentent l'hydrogène.

**10.** Procédé selon l'une des revendications 1 à 9, dans lequel $R_1$ et $R_2$ sont choisis dans le groupe consistant en l'hydrogène et des radicals alkyle contenant 1 à 3 atomes de carbone.

**11.** Procédé selon l'une des revendications 1 à 10, dans lequel $R_1$ et $R_2$ représentent l'hydrogène.

**12.** Procédé selon l'une des revendications 1 à 10, dans lequel l'acide carboxylique est l'acide formique et le réactant est l'urée.

**13.** Procédé selon l'une des revendications 1 à 12, comprenant en plus la réaction entre l'acide carboxylique et le réactant à environ la température ambiante et en agitant jusqu' à le mélange réactionnel est clair.

**14.** Composition pour améliorer la croissance d'une plante, la composition comprenant en tant que substance active le N,N'-diformyl-urée étant préparé en utilisant le procédé selon l'une des revendications 1 à 13 et ayant la formule:

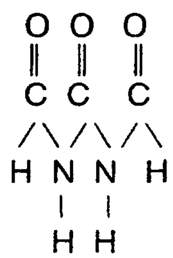

**15.** Utilisation de la composition selon la revendication 14 dans un procédé pour améliorer la croissance d'une plante, dans laquelle la composition est appliquée aux semences pour la plante avant l'implantation, à la terre entourant la plante ou au feuillage de la plante.

**16.** Utilisation selon la revendication 15, dans laquelle la composition est appliquée aux semences de la plante sous forme d'une solution aqueuse contenant le N,N'-diformyl-urée en une concentration entre 0,001 et 1,0 molaire dans une quantité entre 15 et 750 ml de la solution pour 454 kg des semences.

**17.** Utilisation selon la revendication 16, dans laquelle les semences sont plongées dans la solution pour 2 à 48 h avant l'implantation.

**18.** Utilisation selon la revendication 15, dans laquelle la composition est appliquée au feuillage de la plante sous forme d'une solution aqueuse contenant le N,N'-diformule-urée en une concentration entre 0,0001 et 1,0 molaire dans une quantité entre 1 et 100 g du N,N'-diformule-urée pour 4047 $m^2$.

**19.** Utilisation selon l'une des revendications 16 à 18, dans laquelle la solution comprend en plus une huile végétale ou un tensioactif.

**20.** Utilisation selon la revendication 19, dans laquelle l'huile végétale est choisie dans le groupe consistant en l'huile de tournesol et l'huile de soya et le tensioactif est dérivé du groupe des polyphosphates organiques et des nonaphénols éthoxylés.

**21.** Semences traitées pour une plante contenant le N,N'-diformyle-urée de la formule suivanté appliquée à les semences:

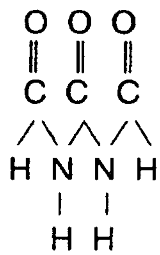

**22.** Semences traitées pour une plante selon la revendication 21, qui ont été plongées dans une solution aqueuse contenant le N,N'-diformule-urée.

**23.** Diformyl-urée ayant la formule